Europäisches Patentamt

European Patent Office

Office européen des brevets

(19)

(11) Veröffentlichungsnummer: **0 295 591**
**A2**

(12) **EUROPÄISCHE PATENTANMELDUNG**

(21) Anmeldenummer: 88109327.2

(22) Anmeldetag: 11.06.88

(51) Int. Cl.⁴: **C07C 51/42 , C07C 55/02**

(30) Priorität: 19.06.87 DE 3720261

(43) Veröffentlichungstag der Anmeldung:
21.12.88 Patentblatt 88/51

(84) Benannte Vertragsstaaten:
AT BE CH DE ES FR GB IT LI NL

(71) Anmelder: Henkel Kommanditgesellschaft auf
Aktien
Postfach 1100 Henkelstrasse 67
D-4000 Düsseldorf-Holthausen(DE)

(72) Erfinder: Waldhoff, Heinrich, Dr.
Am Alten Broich 83 b
D-4018 Langenfeld(DE)
Erfinder: Schindler, Joachim, Dr.
Am Eichelkamp 158
D-4010 Hilden(DE)
Erfinder: Viehweg, Holger, Dr.
Weissenstein 83
D-4018 Langenfeld(DE)

(54) Verfahren zur Trennung von Dicarbonsäuren.

(57) Es wird ein Verfahren zur Trennung von Mischungen von Dicarbonsäuren beschrieben, dadurch gekennzeichnet, daß eine wäßrige Lösung der Mischungen einer Membranfiltration unterworfen wird, wobei der pH-Wert der Lösung so eingestellt wird, daß die Membran bezüglich mindestens einer Komponente der Dicarbonsäuremischung für weniger als 5 % der Stoffmenge permeierbar ist.

EP 0 295 591 A2

"Verfahren zur Trennung von Dicarbonsäuren"

Die Erfindung betrifft ein Verfahren zur Trennung von Dicarbonsäuren mit Hilfe Membranfiltration.

Klassische Trennverfahren für Mischungen von Dicarbonsäuren sind Kristallisation, Rektifikation und Extraktion. Einer Anwendung dieser Verfahren im technischen Maßstab sind aber häufig enge Grenzen gesetzt, da sich die zu trennenden Dicarbonsäuren hinsichtlich der für diese Verfahren relevanten physikalischen Eigenschaften nur wenig unterscheiden. In diesen Fällen ist eine Trennung vielfach nur sehr unvollständig und mit großem Aufwand durchführbar. Dies ist immer dann gegeben, wenn sich größere Carbonsäuremoleküle lediglich hinsichtlich einer funktionellen Gruppe oder eines anderen vergleichbaren Molekülparameters unterscheiden.

Die deutschen Offenlegungsschriften 21 40 133 und 28 53 847 beschreiben Verfahren zur Herstellung von Dicarbonsäuren durch Fermentation bzw. deren Reinigung, wobei die entstehende Mischung der Dicarbonsäuren lediglich zur analytischen Charakterisierung in Form ihrer Methylester auf chromatographischem Wege getrennt wird. Ein ähnliches fermentatives Verfahren beschreibt die DE-OS 21 64 626. Die entstehenden Gemische aus Hydroxy-, Keto- und Dicarbonsäuren werden durch Destillation der Alkylester bei vermindertem Druck getrennt.

In der deutschen Offenlegungsschrift 29 51 177 wird die Reinigung von auf fermentativem Wege hergestellten Dicarbonsäuren durch Umkristallisation aus einem Lösungsmittel beschrieben. Auch die fraktionierte Kristallisation ist als Verfahren zur Trennung von Dicarbonsäuregemischen bekannt.

Die Membranfiltration wird üblicherweise zur Trennung von Stoffen nach Molmasse eingesetzt.

Andererseits ist aus Process Biochemistry, March/ April 1983, S. 8 - 12 bekannt, daß die Durchlässigkeit von Membranen für bestimmte gelöste Stoffe durch die Wahl des pH-Werts der zu filtrierenden Lösung gesteuert werden kann. Beispielsweise lassen sich mit Hilfe einer Spezialmembran Essigsäure bzw. Milchsäure in wäßriger Lösung aufkonzentrieren. Ein Verfahren zur gezielten Stofftrennung von Carbonsäuren und speziell Dicarbonsäuren läßt sich aus dieser Literaturstelle jedoch nicht entnehmen.

Die genannten Verfahren zur Trennung von Mischungen von Dicarbonsäuren, die sich in ihrer chemischen Struktur nur wenig unterscheiden, eignen sich im wesentlichen nur für Labormengen.

Der Erfindung liegt daher die Aufgabe zugrunde, ein Trennverfahren für Dicarbonsäuren zur Verfügung zu stellen, das eine hohe Trennleistung mit der Möglichkeit der Erweiterung der Kapazität verbindet und auch in größerem Maßstab einfach durchzuführen ist.

Gegenstand der Erfindung ist somit ein Verfahren zur Trennung von Mischungen von Dicarbonsäuren, dadurch gekennzeichnet, daß eine wäßrige Lösung der Mischungen einer Membranfiltration unterworfen wird, wobei der pH-Wert der Lösung so eingestellt wird, daß die Membran bezüglich mindestens einer Komponente der Stoffmischung für weniger als 5 % der Stoffmenge permeierbar ist.

Der Erfindung liegt die Erkenntnis zugrunde, daß die Permeabilität der Membranen für Dicarbonsäuren in Abhängigkeit vom pH-Wert der zu filtrierenden Lösung sehr stark von den spezifischen molekularen Eigenschaften der Säure abhängt. So läßt sich beispielsweise bei Vorhandensein einer einzigen Doppelbindung im Molekül einer von einer Fettsäure abgeleiteten Dicarbonsäure die Durchlässigkeit der Membran für dieses Molekül im Vergleich zu gesättigten Verbindungen über den pH-Wert der Lösung so stark beeinflussen, daß das Verfahren für eine technische Trennung solcher Mischungen anwendbar ist.

Das erfindungsgemäße Verfahren dient daher in einer bevorzugten Ausführungsform zur Trennung von Mischungen von gesättigten und ungesättigten Dicarbonsäuren, vorzugsweise alpha-omega-Dicarbonsäuren, insbesondere mit 8 - 24 Kohlenstoffatomen, wobei durch die Wahl des pH-Wertes die Membran für mindestens 95 % der Stoffmenge der gesättigten Dicarbonsäure undurchdringbar gemacht wird.

Dabei können die Komponenten der zu trennenden Mischungen gesättigte und olefinisch ein- bis dreifach ungesättigte, verzweigte oder unverzweigte, gegebenenfalls weitere Substituenten tragende Dicarbonsäuren sein. Beispiele für diese Verbindungen sind die Octandisäure, Nonandisäure, Decandisäure, Undecandisäure, Dodecandisäure, Tridecandisäure, Tetradecandisäure, Pentadecandisäure, Hexadecandisäure, Heptadecandisäure, Octadecandisäure, Nonadecandisäure, Eicosandisäure, Heneicosandisäure, Docosandisäure, Trieicosandisäure und Tetraeicosandisäure sowie die ana logen 1- bis 3-fach olefinisch ungesättigten alpha-omega-Dicarbonsäuren. In allen genannten Fällen können die olefinisch ungesättigten Dicarbonsäuren auch in vollständig oder teilweise epoxidierter Form vorliegen.

Vorzugsweise eignet sich das Verfahren zur Trennung von Mischungen, die durch terminale Oxidation von Alkanen, primären Alkoholen, Monocarbonsäuren oder Monocarbonsäureestern mit Hilfe von Mikroorganismen entstehen, zum Beispiel gemäß der deutschen Patentanmeldung P 35 40 834. Bei diesen Umsetzungen entstehen Gemische, die in ihrer Zusammensetzung den Ausgangsstoffen hinsichtlich Kettenlänge sowie Anzahl und Lage der Doppelbindungen entsprechen können.

Beispielsweise kann man gesättigte, unverzweigte alpha-omega-Dicarbonsäuren von ihren 1- bzw. 2-fach ungesättigten Analoga abtrennen.

Falls die Mischungen der Dicarbonsäuren noch nicht in wäßriger Lösung vorliegen, so werden sie zur Durchführung des Verfahrens zunächst in wäßrige Lösung gebracht. Dabei können mit Wasser mischbare organische Lösungsmittel, wie z.B. Ethanol, Propanol, Isopropanol, Aceton mitverwendet werden. Der pH-Wert der Lösung kann mit üblichen alkalisch reagierenden Stoffen, z.B. Alkalihydroxiden oder Ammoniak eingestellt werden. Zur Wahl des optimalen pH-Wertes ist in Vorversuchen die Abhängigkeit der Trennleistung vom pH-Wert der zu filtrierenden Lösung zu ermitteln. Der Fachmann wird dazu das erfindungsgemäß vorgeschlagene Verfahren bei einer Reihe von pH-Werten durchführen und das Filtrat zur Ermittlung der Trennleistung analytisch charakterisieren. Für die Trennung von Dicarbonsäuren wurden im Bereich pH 4 bis pH 11 gute Trennleistungen gefunden. Insbesondere kann in einem pH-Bereich von 6,5 bis 9,5 gearbeitet werden. Ganz besonders günstige Ergebnisse wurden bei pH-Werten zwischen 8 und 9,5 gefunden.

Für das erfindungsgemäße Verfahren können Membranen aus Celluloseacetat, Derivaten des Celluloseacetats wie Celluloseacetatbutyrat sowie synthetischen Polymeren, wie Polyimiden und Polyamiden, verwendet werden. In einer bevorzugten Ausführungsform des erfindungsgemäßen Verfahrens werden Membranen aus Celluloseacetat verwendet. Die Porenweiten der Membranen, die für das erfindungsgemäße Verfahren verwendet werden, liegen unterhalb von 0,5 µm, wobei Porenweiten kleiner 0,25 µm bevorzugt werden. In dieser bevorzugten Ausführungsform liegt die Untergrenze der Porenweiten der verwendeten Membranen bei 0,1 µm. Neben diesen Membranen, wie sie für die Mikrofiltration üblich sind (Porengrößen im Bereich von einigen Zehntel-µm) sind auch solche Membranen für das erfindungsgemäße Verfahren anwendbar, die für die Ultrafiltration eingesetzt werden. In diesem Fall sind Porengrößen verwendbar, die zur Abtrennung von Substanzen mit Molmassen größer 500 Dalton dienen.

Die Temperatur, bei der die Membranfiltration durchgeführt wird, bestimmt sich aus der Viskosität der Lösung und den Eigenschaften der gewählten Materialien. Der zur Verfügung stehende Temperaturbereich wird durch den Gefrierpunkt der zu filtrierenden Lösung einerseits, und durch die Destabilisierung der Membran bei hohen Temperaturen andererseits begrenzt.

Der Fachmann wird aus Gründen der Energieersparnis bevorzugt bei Raumtemperatur arbeiten. Andererseits sinkt mit steigender Temperatur die Viskosität der Lösung, so daß bei höheren Temperaturen größere Durchsätze erzielt werden können. Der zur Verfügung stehende Temperaturbereich liegt üblicherweise zwischen etwa 0 °C und etwa 120 °C, vorzugsweise zwischen 20 °C und 60 °C.

Ein weiterer Parameter des erfindungsgemäßen Verfahrens ist der auf die Lösung ausgeübte äußere Druck. Durch eine Erhöhung des Druckes kann der Mengendurchsatz bei einer Membranfiltration entscheidend erhöht werden. So kann ein zusätzlicher äußerer Druck bis zu 20 bar auf die Lösung ausgeübt werden. In einer besonders bevorzugten Ausführungsform des erfindungsgemäßen Verfahrens wird ein zusätzlicher äußerer Druck bis 5 bar ausgeübt.

Die Vorteile des erfindungsgemäßen Verfahrens bestehen darin, daß es möglich ist, einzelne Komponenten von Dicarbonsäuremischungen, die sich hinsichtlich ihrer molekularen Eigenschaften nur wenig unterscheiden, im technischen Maßstab mit vertretbarem Aufwand weitgehend, d.h. zu mindestens 95 % abzutrennen. Dadurch ist es möglich, unerwünschte Nebenprodukte für eine weitere Verarbeitung der hergestellten Dicarbonsäuren abzutrennen.

Die Vorteile und die Leistungsfähigkeit des erfindungsgemäßen Verfahrens zeigen sich im folgenden Beispiel.

## Beispiel

10 ml einer Fermenterlösung, die ca. 10 g/l $C_{16}$-alpha-omega-Dicarbonsäuren enthält, davon ca. 2 g ungesättigte Spezies, wurden mit Natronlauge auf einen pH-Wert von 9 eingestellt. Die Lösung wurde durch einen Membranfilter Satorius Minisart NML mit einem Porendurchmesser von 0,2 µm filtriert. Die Aufarbeitung des Filtrats erfolgte durch Festflüssigextraktion mit Sep-pak-Kartuschen (Sep-pak RP18, Firma Millipore). Die Analyse des Filtrats erfolgte gaschromatographisch bzw. mit HPLC. Im Chromatogramm der filtrierten Probe wurde für die gesättigte $C_{16}$-alpha-omega-Dicarbonsäure eine Eliminationsrate größer 95 % gefunden.

## Ansprüche

1. Verfahren zur Trennung von Mischungen von Dicarbonsäuren, dadurch gekennzeichnet, daß eine wäßrige Lösung der Mischungen einer Membranfiltration unterworfen wird, wobei der pH-Wert der Lösung so eingestellt wird, daß die Membran bezüglich mindestens einer Komponente der Dicarbonsäuremischung für weniger als 5 % der Stoffmenge permeierbar ist.

2. Verfahren nach Anspruch 1, dadurch gekennzeichnet, daß der pH-Wert der wäßrigen Lösung in Abhängigkeit von der Permeabilität der abzutrennenden Komponente auf einen Wert zwischen 4 und 11, vorzugsweise zwischen 6,5 und 9,5, eingestellt wird.

3. Verfahren nach einem der Ansprüche 1 und 2, dadurch gekennzeichnet, daß zur Trennung Membranen aus Celluloseestern wie Celluloseacetat und Celluloseacetatbutyrat, oder synthetischen Polymeren wie Polyamid oder Polyimid, vorzugsweise Celluloseacetat, verwendet werden.

4. Verfahren nach einem der Ansprüche 1 - 3, dadurch gekennzeichnet, daß die Porenweite der Membran kleiner als 0,5 μm, vorzugsweise kleiner als 0,25 μm ist, wobei die Untergrenze eine Ausschlußmolekularmasse von 500 Dalton darstellt.

5. Verfahren nach einem der Ansprüche 1 bis 4, dadurch gekennzeichnet, daß man Mischungen von alpha-omega-Dicarbonsäuren mit 8 - 24 C-Atomen trennt.

6. Verfahren nach einem der Ansprüche 1 - 5, dadurch gekennzeichnet, daß man gesättigte Dicarbonsäuren von ungesättigten Dicarbonsäuren trennt.

7. Verfahren nach einem der Ansprüche 1 - 6, dadurch gekennzeichnet, daß Mischungen eingesetzt werden, die unverzweigte alpha-omega-Dicarbonsäuren und ungesättigte, unverzweigte alpha-omega-Dicarbonsäuren mit 1 - 3 Doppelbindungen enthalten können.

8. Verfahren nach einem der Ansprüche 1 - 7, dadurch gekennzeichnet, daß zur Einstellung des pH-Wertes Alkalihydroxide oder Ammoniak verwendet werden.

9. Verfahren nach einem der Ansprüche 1 - 8, dadurch gekennzeichnet, daß man bei Temperaturen zwischen dem Gefrierpunkt der Lösung und der Stabilitätsgrenze der Membran, und vorzugsweise bei 20 - 60°C arbeitet, und daß ein äußerer Druck bis zu 20 bar, vorzugsweise bis zu 5 bar, eingestellt wird.